# EUROPEAN PATENT SPECIFICATION

(11) **EP 1 631 266 B1**
(45) Date of publication and mention of the grant of the patent: **16.03.2011**
(21) Application number: 04776014.5
(22) Date of filing: 13.05.2004
(51) Int. Cl.: A61K 31/00, A61K 31/4439, A61K 38/00, A61K 38/48, A61K 9/00, A61P 19/00

(54) **A METHOD OF TREATING DEGENERATIVE DISC DISEASE**
VERFAHREN ZUR BEHANDLUNG VON DEGENERATIVER BANDSCHEIBENERKRANKUNG
METHODE DE TRAITEMENT D'UNE MALADIE DISCALE DEGENERATIVE

(30) Priority: 13.05.2003 US 470098 P; 06.06.2003 US 456948; 30.06.2003 US 610355; 31.07.2003 US 631487; 13.08.2003 US 640412
(43) Date of publication of application: 08.03.2006
(60) Divisional application: 10183305.1
(73) Proprietor: DePuy Spine, Inc., Raynham, MA 02767-0350 (US)
(72) Inventor: SERHAN, Hassan, South Easton, MA 02375 (US); DIMAURO, Thomas, M., Southborough, MA 01772 (US); ATTAWIA, Mohamed, Canton, MA 02021 (US); GRACE, Melissa, Raynham, MA 02767 (US); KADIYALA, Sudhakar, Newton, MA 02459 (US); URBAHNS, David, Barrington, RI 02806 (US); BRUDER, Scott, Sudbury, MA 01776 (US); COLLINS, Gregory, East Sandwich, MA 02537 (US); BROWN, Laura, J., Hamilton Square, NJ 08690 (US); GEESIN, Jeff, Doylestown, PA 18901 (US); PLOUHAR, Pamela, L., South Bend, IN 46614 (US); SMITH, Catherine, East Falmouth, MA 02536 (US); SIEKIERKA, John, Towaco, NJ 07082 (US)
(74) Representative: Belcher, Simon James
(86) International application number: PCT/US2004/015285
(87) International publication number: WO 2005/000283

(56) References cited:
- US-A1- 2001 006 948
- US-A1- 2001 026 801
- US-A1- 2002 032 155
- US-A1- 2002 198 599
- "Abstracts of the North American Spine Society 17th Annual Meeting. Montreal, Canada, October 29-November 2, 2002." THE SPINE JOURNAL : OFFICIAL JOURNAL OF THE NORTH AMERICAN SPINE SOCIETY. 2002 SEP-OCT, vol. 2, no. 5 Suppl, September 2002 (2002-09), pages 49S-50S, XP002317026 ISSN: 1529-9430
- TOBINICK EDWARD L: "Targeted etanercept for discogenic neck pain: Uncontrolled, open-label results in two adults." CLINICAL THERAPEUTICS, vol. 25, no. 4, April 2003 (2003-04), pages 1211-1218, XP001205176 ISSN: 0149-2918
- ALINI M ET AL: "A biological approach to treating disc degeneration: Not for today, but maybe for tomorrow" EUROPEAN SPINE JOURNAL, SPRINGER VERLAG, BERLIN, DE, vol. 11, no. SUPPL 2, 2002, pages S215-S220, XP002287478 ISSN: 0940-6719

## Description

### BACKGROUND TO THE INVENTION

The natural intervertebral disc contains a jelly-like nucleus pulposus surrounded by a fibrous annulus fibrosus. Under an axial load, the nucleus pulposus compresses and radially transfers that load to the annulus fibrosus. The laminated nature of the annulus fibrosus provides it with a high tensile strength and so allows it to expand radially in response to this transferred load.

In a healthy intervertebral disc, cells within the nucleus pulposus produce an extracellular matrix (ECM) containing a high percentage of proteoglycans. These proteoglycans contain sulfated functional groups that retain water, thereby providing the nucleus pulposus with its cushioning qualities. These nucleus pulposus cells may also secrete small amounts of cytokines as well as matrix metalloproteinases (MMPs). These cytokines and MMPs help regulate the metabolism of the nucleus pulposus cells.

In some instances of degenerative disc disease (DDD), gradual degeneration of the intervertebral disc is caused by mechanical instabilities in other portions of the spine. In these instances, increased loads and pressures on the nucleus pulposus cause the cells within the disc (or invading macrophages) to emit larger than normal amounts of the above-mentioned cytokines. In other instances of DDD, genetic factors or apoptosis can also cause the cells within the nucleus pulposus to emit toxic amounts of these cytokines and MMPs. In some instances, the pumping action of the disc may malfunction (due to, for example, a decrease in the proteoglycan concentration within the nucleus pulposus), thereby retarding the flow of nutrients into the disc as well as the flow of waste products out of the disc. This reduced capacity to eliminate waste may result in the accumulation of high levels of proinflammatory cytokines and/or MMPs that may cause nerve irritation and pain.

As DDD progresses, toxic levels of the cytokines and MMPs present in the nucleus pulposus begin to degrade the extracellular matrix. In particular, the MMPs (as mediated by the cytokines) begin cleaving the water-retaining portions of the proteoglycans, thereby reducing their water-retaining capabilities. This degradation leads to a less flexible nucleus pulposus, and so changes the loading pattern within the disc, thereby possibly causing delamination of the annulus fibrosus. These changes cause more mechanical instability, thereby causing the cells to emit even more cytokines, typically thereby upregulating MMPs. As this destructive cascade continues and DDD further progresses, the disc begins to bulge ("a herniated disc"), and then ultimately ruptures, causing the nucleus pulposus to contact the spinal cord and produce pain.

US-A-2001/006948 discloses techniques for introducing a gene into an intervertebral disc cell. A soluble TNF-α receptor can be used to bind TNF-α so as to prevent it from having a damaging effect on the disc matrix.

US-A-2001/0026801 discloses a method for inhibiting the action of TNF for treating nerve root injury caused by a herniated nucleus pulposus by administering a TNF antagonist such as etanercept or infliximab intralesionally.

### SUMMARY OF THE INVENTION

The invention provides a formulation for use in a method of treating degenerative disc disease, as defined in claim 1.

As used herein, an antagonist refers to an inhibitor of a cytokine protein or proteins emitted from nucleus pulposus and/or annulus fibrosus cells. Such proteins can be emitted in toxic amounts as a result of DDD.

There are believed to be several advantages to directly transdiscally administering such antagonists to a targeted disc:

First, HSCAs inhibit the activity of cytokines. Since it is known that cytokines (such as interleukins and TNF-α) play primary roles in the degradation of the extracellular matrix (ECM) of the nucleus pulposus, injecting an antagonist directly into the disc in a therapeutically effective amount can prevent the cytokines from causing any further ECM degradation. In effect, the transdiscal adminstration of the antagonists helps arrest the aging process of the degenerating disc. Accordingly, the present invention seeks to treat the degenerative disc at a much earlier stage of DDD and thereby prevents degradation of the ECM.

Second, some HSCAs inhibit the activity of TNF-α. It is further known that nerve ending nociceptors are present within the annulus fibrosus, and that TNF-α participates in the irritation of nerves. It is believed that injecting a TNF-α antagonist into the disc space also prevents the TNF-α from causing nerve irritation within the disc. Thus, the pain attributed to irritation of these nerves can be efficiently eliminated.

Third, since some antagonists are specific, they do not inhibit non-targeted cells, tissue or proteins. In addition, the antagonist may be combined with other (i.e., additional) therapeutic agents (such as growth factors) that can also be injected into the disc without reducing the effectiveness of those agents.

Fourth, since the annulus fibrosus portion of the disc comprises a dense fibrosus structure, this outer component of the disc may provide a suitable depot for the antagonist, thereby increasing its half-life in the disc.

In one embodiment, the antagonist is administered in a dosage to produce a local tissue concentration of between about 5 µg/kg and about 50 µg/kg. In one embodiment, the formulation is administered in an amount of less than about 1 cc. In one embodiment, the antagonist is present in the formulation in an amount of at least about 100 mg/ml. In one embodiment, the antagonist is present in the formulation in an amount of no more than about 0.5 mg.

The formulation further comprises a sustained release device comprising a hydrogel. In one embodiment, the sustained release device provides controlled release. In another embodiment, the sustained release device provides continuous release. In yet another embodiment, the sustained release device provides intermittent release.

In one embodiment, the sustained release device comprises a biosensor. In one embodiment, the sustained release device comprises a plurality of microspheres. In one embodiment, the sustained release device comprises an inflammatory-responsive delivery system.

In another embodiment, the antagonist is predominantly released from the sustained delivery device by its diffusion through the sustained delivery device. In yet another embodiment, the sustained delivery device is a polymer. In one embodiment, the antagonist is predominantly released from the sustained delivery device by biodegradation of the sustained delivery device.

In one embodiment, the formulation further comprises at least one additional (e.g., a second, third or fourth) therapeutic agent. In one embodiment, at least one additional therapeutic agent is co-administered (for example, sequentially or simultaneously) with the antagonist. In one embodiment, the formulation is administered in an amount effective to reduce pain. In one embodiment, the formulation is administered in an amount effective to inhibit degradation of an extracellular matrix of the nucleus pulposus. The additional therapeutic agent can comprise any additional agent disclosed herein, including any antagonist disclosed herein.

In one embodiment, the additional therapeutic agent is a growth factor. The growth factor can be present in an amount effective to repair disc tissue. In one embodiment, the growth factor is a TGF-β. In one embodiment, the growth factor is provided by platelet concentrate.

In one embodiment, the additional therapeutic agent is plasmid DNA.

In one embodiment, the nucleus pulposus is removed prior to transdiscally administering the formulation.

In one embodiment, the formulation is administered through a drug pump. In yet another embodiment, the formulation is administered through a needle. In one embodiment, the needle has a maximum gauge of 24 gauge. In yet another embodiment, the formulation is administered in a volume of between about 0.03 ml and about 0.3 ml.

In one embodiment, the degenerating disc is an intact disc. In another embodiment, the degenerating disc is a ruptured disc. In one embodiment, the degenerating disc is delaminated. In one embodiment, the degenerating disc has fissures.

The proinflammatory protein is TNF-α. In one embodiment, the predetermined level for TNF-α is at least 20 pg/ml. In one embodiment, the predetermined level for TNF-α is at least 30 pg/ml. In one embodiment, the predetermined level for TNF-α is at least 1000 pg/disc.

The formulation can be used in a method of preventing degeneration of an intervertebral disc in a human individual, comprising:
a) determining a genetic profile of the individual;
b) comparing the profile of the individual against a pre-determined genetic profile level of at-risk humans;
c) determining that the individual is an at-risk patient; and
d) injecting the formulation of the invention into a disc of the individual.

### DETAILED DESCRIPTION OF THE INVENTION

For the purposes of the present invention, the terms "inhibitor" and "antagonist" are used interchangeably. A protein may be inhibited at the synthesis level, at the translation level, by shedding, by antibodies, or by soluble receptors. The term "patient" refers to a human having a degenerating disc. As used herein, antagonists include, for example, inhibitors of substances from a disc or present in a disc in patients with DDD.

For the purposes of the present invention "transdiscal administration" involves injecting a formulation into the nucleus pulposus of a degenerating disc, such as a relatively intact degenerating disc.

Because DDD is a continuous process, the degenerating disc to which the therapeutic drug is administered may be in any one of a number of degenerative states. Accordingly, the degenerating disc may be an intact disc. The degenerating disc may be a herniated disc (*i.e*., wherein a portion of the annulus fibrosus has a bulge). The degenerating disc may be a ruptured disc (*i.e*., wherein the annulus fibrosus has ruptured and the bulk nucleus pulposus has exuded). The degenerating disc may be delaminated (*i.e*., wherein adjacent layers of the annulus fibrosus have separated). The degenerating disc may have fissures (*i.e*., wherein the annulus fibrosus has fine cracks or tears through which selected molecules from the nucleus pulposus can leak). In all of these degenerative states, the extra-cellular matrix of either the annulus fibrosus or nucleus pulposus is also degrading.

US Published Patent Application No. US 2003/0039651 ("Olmarker I") teaches a therapeutic treatment of nerve disorders comprising administration of a therapeutically effective dosage of compounds, including inhibitors of MMPs, at least two substances selected from the group consisting of TNF inhibitors (both specific and non-specific), IL-1 inhibitors, IL-6 inhibitors, IL-8 inhibitors, FAS inhibitors, FAS ligand inhibitors, and IFN-gamma inhibitors.

In the examples of Olmarker I, it is taught that the therapeutic compounds are to be administered through systemic pathways. In particular, Olmarker I teaches that "the major contribution of TNF-alpha may be derived from recruited, aggregated and maybe even extravasated leukocytes, and that successful pharmacologic block may be achieved only by systemic treatment." Of note, Olmarker I appears to discourage the local addition of at least one therapeutic compound (doxycycline) to an autotransplanted nucleus pulposus to be applied to a spinal cord.

PCT Published Patent Application No. WO 02/100387 ("Olmarker II") teaches the prevention of neovascularization and/or neo-innervation of intervertebral discs by the administration of anti-angiogenic substances. Again, however, Olmarker II teaches systemic administration of these therapeutic agents.

U.S. Patent No. 6,419,944 ("Tobinick") discloses treating herniated discs with cytokine antagonists. Tobinick teaches that local administration involves a subcutaneous injection near the spinal cord. Accordingly, Tobinick does not teach a procedure involving a sustained delivery of a drug for the treatment of DDD, nor directly administering a drug into the disc.

U.S. Published Patent Application No. 2003/0049256 ("Tobinick II") discloses that injection of such therapeutic molecules to the anatomic area adjacent to the spine is accomplished by interspinous injection, and preferably is accomplished by injection through the skin in the anatomic area between two adjacent spinous processes of the vertebral column.

Tobinick II further teaches that the therapeutic compounds may be administered by interspinous injection in the human and that the dosage level is in the range of 1 mg to 300 mg per dose, with dosage intervals as short as two days. Tobinick II further discloses that other therapeutic compounds are administered in a therapeutically effective dose, which will generally be 10 mg to 200 mg per dose, and their dosage interval will be as short as once daily.

Tobinick, Swiss Med Weekly, 133: 170-7 (2003) ("Tobinick III") teaches perispinal and epidural administration of TNF inhibitors.

Karppinen, Spine, 28(8): 750-4 (2003), teaches intravenously injecting or orally administering infliximab into patients suffering from sciatica.

As with Tobinick I and II, Karppinen does not teach a procedure involving a sustained delivery of a drug for the treatment of DDD, nor directly administering a drug into the disc space.

U.S. Patent No. 6,352,557 (Ferree) teaches adding therapeutic substances such as anti-inflammatory medications to morselized extra-cellular matrix, and injecting that combination into an intervertebral disc. However many anti-inflammatory agents are non-specific and, therefore, may produce unwanted side effects upon other cells, proteins and tissue. In addition, the pain-reducing effect of these agents is typically only temporary. Lastly, these agents typically only relieve pain, and are neither curative nor restorative.

Alini, Eur. Spine J., II(Supp.2): S215-220 (2002), teaches therapies for early stage DDD, including injection of inhibitors of proteolytic enzymes or biological factors that stimulate cell metabolic activity (*i*.*e*., growth factors) in order to slow down the degenerative process. Inhibitors of proteolytic enzymes constitute a broad class of compounds, including i) inhibitors of proteolytic enzyme synthesis and ii) inhibitors of proteolytic enzyme activity. Alini I does not specify any desired types of inhibitors of proteolytic enzymes.

U.S. Published Patent Application US 2002/0026244 ("Trieu") discloses an intervertebral disc nucleus comprising a hydrogel that may deliver desired pharmacological agents. Trieu teaches that these pharmacological agents may include growth factors such as TGF-β and anti-inflammatory drugs, including steroids. Trieu further teaches that these pharmacological agents may be dispersed within the hydrogel having an appropriate level of porosity to release the pharmacological agent at a desired rate. Trieu teaches that these agents may be released upon cyclic loading or upon resorption.

Goupille, Spine, 23(14): 1612-1626 (1998) identifies Tissue Inhibitors of MMPs ("TIMPs") as degraders of MMP activity. Goupille reports that TIMP-1 and TIMP-2 bind noncovalently to active MMPs in a 1:1 molar ratio and specifically inhibit their enzymatic activity. However, Goupille also identifies corticosteroids, retinoic acid, TGF-β, PGE1 and PGE2 as inhibitors of MMP synthesis; identifies α2-macroglobulin, hydroxamic acid, derivatives, tetracyclines and quinolones as inhibitors of MMP activity, and identifies bFGF, EGF, Retenoic acid, TGF-β, IL-6, IL-1 LIF, dexamethasone, phorbol ester, and synthetic Vitamin A analogs as stimulators of TIMPs. Moreover, as to administration route, Goupille explicitly identifies only the oral administration route.

Takegami, Spine, 27(12):1318-25 (2000) teaches that injecting TGF-β into the disc space results in enhanced replenishment of the extracellular matrix damaged by cytokines. Takegami further teaches that the half-life of a growth factor injected into the intervertebral disc can be expected to be longer than that injected into a synovial joint because the nucleus pulposus is surrounded by the fibrous structure of the annulus fibrosus, thus providing a confined environment. Diwan, Tissue Engineering in Orthopedic Surgery, 31(3):453-464 (2000), reports on another Takegami paper that concluded that a delivery system allowing prolonged delivery (>3 days) would have to be used to obtain the observed effect of the growth factor.

Alini, Spine 28(5):446-54 (2003), discloses a cell seeded collagen-hyaluronan scaffold supplemented with growth factors such as TNF-β, bFGF, and IGF-1 for use in regenerating a nucleus pulposus.

Maeda et al. Spine 25(2):166-169 (2000) reports on the *in vitro* response to interleukin-1 receptor antagonist protein (IRAP) of rabbit annulus fibrosus exposed to IL-1. Maeda suggests that IRAP could be useful in inhibiting the degradation of the disc.

Yabuki, Spine, 26(8):870-5 (2001), teaches the use of an anti-TNF drug for the treatment of sciatica.

U.S. Patent No. 6,277,969 ("Le") discloses the use of anti-TNF antibodies for therapy of TNF-mediated pathologies. Le teaches parenteral administration of the antibodies.

Ariga, Spine, 28(14):1528-33 (2003), reports that the application of a p38 MAP kinase inhibitor to a culture of organ-cultured intervertebral discs increased the occurrence of apoptosis in endplate chondrocytes in the culture.

Olmarker, Spine, 26(8): 863-9 (2001) ("Olmarker I") and Aoki, Spine, 27(15): 1614-17 (2002) teach that TNF-α (TNF-alpha) appears to play a role in producing the pain associated with the nucleus pulposus contacting nerve roots of the spinal cord. Olmarker discourages the use of non-specific TNF-α inhibitors in spine-related drug therapies, and particularly those that have anti-biotic effects.

U.S. Published Patent Application No. U.S. 2003/0039651 ("Olmarker II") teaches a therapeutic treatment of nerve disorders comprising administration of a therapeutically effective dosage of a number of drugs, including cycline compounds. According to Olmarker, tetracycline are non-specific inhibitors of TNF-α.

In the examples of Olmarker II, Olmarker II further teaches that these drugs are to be administered through systemic pathways. In particular, Olmarker II teaches that "the major contribution of TNF-α may be derived from recruited, aggregated and maybe even extravasated leukocytes, and that successful pharmacologic block may be achieved only by systemic treatment. Of note, Olmarker II appears to discourage the local addition of doxycycline to an autotransplanted nucleus pulposus to be applied to a spinal cord.

PCT Published Patent Application No. WO 02/100387 ("Olmarker III") teaches the prevention of neovasculariation and/or neo-innervation of intervertebral discs by the administration of anti-angiogenic substances. Again, however, Olmarker III teaches systemic administration of these therapeutic agents.

In sum, although investigators have generally taught the transdiscal administration of inhibitors of proteolytic enzymes, when investigators have specifically identified the administration of a cycline compound for spine-related therapy, the investigators appear to teach only the administration of certain antagonists (e.g., a cycline compound) to tissue outside the disc. Moreover, Olmarker II appears to discourage the administration of a cycline compound to a nucleus pulposus.

### CYTOKINE ANTAGONISTS

The HSCA inhibits the action of a specific pro-inflammatory cytokine released by disc cells or by invading macrophages during the degenerative process.

In some embodiments, the HSCA inhibits the cytokine by preventing its production. In some embodiments, the HSCA inhibits the cytokine by binding to a membrane-bound cytokine. In others, the HSCA inhibits the cytokine by binding to a solubilized, e.g. soluble, cytokine. In some embodiments, the HSCA inhibitor inhibits the cytokine by both binding to membrane bound cytokines and to solubilized cytokines. In some embodiments, the HSCA is a monoclonal antibody ("mAb"). The use of mAbs is highly desirable since they bind specifically to a certain target protein and to no other proteins. In some embodiments, the HSCA inhibits the cytokine by binding to a natural receptor of the target cytokine.

In some embodiments, the HSCA inhibits the cytokine by preventing its production. One example thereof is an inhibitor of p38 mitogen activated protein (MAP) kinase.

In some embodiments, the TNF inhibitor inhibits the TNF by binding to membrane-bound TNF in order to prevent its release from membrane. In others, the TNF inhibitor inhibits the TNF by binding to solubilized TNF. One example thereof is etanercept (ENBREL^{®}, Amgen). In some embodiments, the TNF inhibitor inhibits the TNF by both binding to membrane bound TNF and to solubilized TNF. One example thereof is REMICADE^{®} infliximab. In some embodiments, the HSCA inhibits the cytokine (e.g., TNF-α) by binding to a natural receptor of the target cytokine. In some embodiments, the TNF-α inhibitor is an inhibitor of TNF-α synthesis.

Preferred TNF antagonists include, but are not limited to, the following: etanercept (ENBREL^{®}, Amgen); infliximab (REMICADE^{®}-Johnson and Johnson); D2E7, a human anti-TNF monoclonal antibody (Knoll Pharmaceuticals, Abbott Laboratories); CDP 571 (a humanized anti-TNF IgG4 antibody); CDP 870 (an anti-TNF alpha humanized monoclonal antibody fragment), both from Celltech; soluble TNF receptor Type I (Amgen); pegylated soluble TNF receptor Type I (PEGs TNF-R1) (Amgen); and onercept, a recombinant TNF binding protein (r-TBP-1) (Serono).

TNF antagonists suitable for compositions, combination therapy, coadministration, devices and/or methods of the present invention (optionally further comprising at least one antibody, specified portion and/or variant thereof, of the present invention), include, but are not limited to, anti-TNF antibodies (e.g., at least one TNF antagonist (e.g., but not limited to, a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, REMICADE^{®} infliximab, enteracept (ENBREL^{®}), adalimulab (HUMIRA™), CDP-571, CDP-870, afelimomab, lenercept, and the like), antigen-binding fragments thereof, and receptor molecules which bind specifically to TNF; compounds which prevent and/or inhibit TNF synthesis, TNF release or its action on target cells, such as thalidomide, tenidap, phosphodiesterase inhibitors (e.g. pentoxifylline and rolipram), A2b adenosine receptor agonists and A2b adenosine receptor enhancers; compounds which prevent and/or inhibit TNF receptor signalling, such as mitogen activated protein (MAP) kinase inhibitors; compounds which block and/or inhibit membrane TNF cleavage, such as metalloproteinase inhibitors; compounds which block and/or inhibit TNF activity, such as angiotensin converting enzyme (ACE) inhibitors (e.g., captopril); and compounds which block and/or inhibit TNF production and/or synthesis, such as MAP kinase inhibitors.

As used herein, a "tumor necrosis factor antibody," "TNF antibody," "TNFα antibody," or fragment and the like decreases, blocks, inhibits, abrogates or interferes with TNFα activity *in vitro, in situ* and/or preferably *in vivo.* For example, a suitable TNF human antibody of the present invention can bind TNFα and includes anti-TNF antibodies, antigen-binding fragments thereof, and specified mutants or domains thereof that bind specifically to TNF-alpha (TNFα). A suitable TNF antibody or fragment can also decrease block, abrogate, interfere, prevent and/or inhibit TNF RNA, DNA or protein synthesis, TNF release, TNF receptor signaling, membrane TNF cleavage, TNF activity, TNF production and/or synthesis.

Chimeric antibody cA2 consists of the antigen binding variable region of the high-specificity neutralizing mouse anti-human TNFα IgG1 antibody, designated A2, and the constant regions of a human IgG1, kappa immunoglobulin. The human IgG1 Fc region improves allogeneic antibody effector function, increases the circulating serum half-life and decreases the immunogenicity of the antibody. The avidity and epitope specificity of the chimeric antibody cA2 is derived from the variable region of the murine antibody A2. In a particular embodiment, a preferred source for nucleic acids encoding the variable region of the murine antibody A2 is the A2 hybridoma cell line.

Chimeric A2 (cA2) neutralizes the cytotoxic effect of both natural and recombinant human TNFα in a dose dependent manner. From binding assays of chimeric antibody cA2 and recombinant human TNFα, the specificity constant of chimeric antibody cA2 was calculated to be 1.04x1010M-1. Preferred methods for determining monoclonal antibody specificity and specificity by competitive inhibition can be found in Harlow et al., Antibodies: A Laboratory Manual, Cold Spring Harbor Laboratory Press, Cold Spring Harbor, New York (1988); Colligan et al., eds., Current Protocols in Immunology, Greene Publishing Assoc. and Wiley Interscience, New York, (1992-2000); Kozbor et al., Immunol. Today, 4:72-79 (1983); Ausubel et al., eds. Current Protocols in Molecular Biology, Wiley Interscience, New York (1987-2000); and Muller, Meth Enzymol., 92:589-601 (1983).

In a particular embodiment, murine monoclonal antibody A2 is produced by a cell line designated c134A. Chimeric antibody cA2 is produced by a cell line designated c168A.

Additional examples of monoclonal anti-TNF antibodies that can be used in the present invention are described in the art (see, e.g., U.S. Patent No. 5,231,024; Möller, A. et al., Cytokine 2(3):162-169 (1990); Rathjen et al., International Publication No. WO 91/02078 (published February 21, 1991); Rubin et al., EPO Patent Publication No. 0 218 868 (published April 22, 1987); Yone et al., EPO Patent Publication No. 0 288 088 (October 26, 1988); Liang, et al., Biochem. Biophys. Res. Comm. 137:847-854 (1986); Meager, et al., Hybridoma 6:305-311 (1987); Fendly et al., Hybridoma 6:359-369 (1987); Bringman, et al., Hybridoma 6:489-507 (1987); and Hirai, et al., J. Immunol. Meth. 96:57-62 (1987).

Preferred TNF receptor molecules useful in the present invention are those that bind TNFα with high specificity (*see, e.g.,* Feldmann et al., International Publication No. WO 92/07076 (published April 30, 1992); Schall et al., Cell, 61:361-370 (1990); and Loetscher et al., Cell 61:351-359 (1990) and, optionally, possess low immunogenicity. In particular, the 55 kDa (p55 TNF-R) and the 75 kDa (p75 TNF-R) TNF cell surface receptors are useful in the present invention. Truncated forms of these receptors, comprising the extracellular domains (ECD) of the receptors or functional portions thereof (see, e.g., Corcoran et al., Eur. J. Biochem. 223:831-840 (1994)), are also useful in the present invention. Truncated forms of the TNF receptors, comprising the ECD, have been detected in urine and serum as 30 kDa and 40 kDa TNF inhibitory binding proteins (Engelmann, H. et al., J. Biol. Chem. 265:1531-1536 (1990)). TNF receptor multimeric molecules and TNF immunoreceptor fusion molecules, and derivatives and fragments or portions thereof, are additional examples of TNF receptor molecules which are useful in the methods and compositions of the present invention. The TNF receptor molecules which can be used in the invention are characterized by their ability to treat patients for extended periods with good to excellent alleviation of symptoms and low toxicity. Low immunogenicity and/or high specificity, as well as other undefined properties, can contribute to the therapeutic results achieved.

TNF receptor multimeric molecules useful in the present invention comprise all or a functional portion of the ECD of two or more TNF receptors linked via one or more polypeptide linkers or other nonpeptide linkers, such as polyethylene glycol (PEG). The multimeric molecules can further comprise a signal peptide of a secreted protein to direct expression of the multimeric molecule.

TNF immunoreceptor fusion molecules useful in the methods and compositions of the present invention comprise at least one portion of one or more immunoglobulin molecules and all or a functional portion of one or more TNF receptors. These immunoreceptor fusion molecules can be assembled as monomers, or hetero- or homo-multimers. The immunoreceptor fusion molecules can also be monovalent or multivalent. An example of such a TNF immunoreceptor fusion molecule is TNF receptor/IgG fusion protein. TNF immunoreceptor fusion molecules and methods for their production have been described in the art (Lesslauer et al., Eur. J. Immunol. 21:2883-2886 (1991); Ashkenazi et al., Proc. Natl. Acad. Sci. USA 88:10535-10539 (1991); Peppel et al., J. Exp. Med. 174:1483-1489 (1991); Kolls et al., Proc. Natl. Acad. Sci. USA 91:215-219 (1994); Butler et al., Cytokine 6(6):616-623 (1994); Baker et al., Eur. J. Immunol. 24:2040-2048 (1994); Beutler et al., and U.S. Patent No. 5,447,851. Methods for producing immunoreceptor fusion molecules can also be found in Capon et al., U.S. Patent No. 5,116,964; Capon et al., U.S. Patent No. 5,225,538; and Capon et al., Nature 337:525-531 (1989).

A functional equivalent, derivative, fragment or region of a TNF receptor molecule refers to the portion of the TNF receptor molecule, or the portion of the TNF receptor molecule sequence which encodes the TNF receptor molecule, that is of sufficient size and sequences to functionally resemble TNF receptor molecules that can be used in the present invention (e.g., bind TNFα with high specificity and possess low immunogenicity). A functional equivalent of a TNF receptor molecule also includes modified TNF receptor molecules that functionally resemble TNF receptor molecules that can be used in the present invention (e.g., bind TNFα with high specificity and possess low immunogenicity). For example, a functional equivalent of a TNF receptor molecule can contain a "SILENT" codon or one or more amino acid substitutions, deletions or additions (e.g., substitution of one acidic amino acid for another acidic amino acid; or substitution of one codon encoding the same or different hydrophobic amino acid for another codon encoding a hydrophobic amino acid). See Ausubel et al., Current Protocols in Molecular Biology, Greene Publishing Assoc. and Wiley-Interscience, New York (1987-2003).

In some embodiments, the monoclonal antibody that inhibits TNF-α is selected from the group consisting of monoclonal rodent-human antibodies, rodent antibodies, human antibodies or any portions thereof, having at least one antigen binding region of an immunoglobulin variable region, which antibody binds TNF. Preferably, this monoclonal antibody is selected from the group of compounds disclosed in U.S. Patent No. 6,277,969. In some embodiments, the REMICADE^{®} infliximab is delivered in a formulation having an infliximab concentration of between about 30 mg/ml and about 60 mg/ml.

It is further believed that the formulation of the present invention can also be used to prevent degeneration of an intervertebral disc in a human individual, namely, by following a procedure comprising:
a) determining a genetic profile of the individual;
b) comparing the profile of the individual against a pre-determined genetic profile level of at-risk humans;
c) determining that the individual is an at-risk patient; and
d) injecting an antagonist into a disc of the individual.

### ADMINISTRATION TO PATIENTS WITH DEGENERATIVE DISC DISEASE

DDD involves the progressive degeneration of a disc in which many factors are involved. In many instances, simply providing a single dose or even a regimen over the space of a few days may not be sufficient to resolve the DDD. For example, if DDD were caused in part by mechanical instability in a functional spinal unit, then simply providing a one-time therapy for the transdiscal cells would likely only delay the onset of the DDD. Therefore, there is a need to provide a long-term drug therapy treatment of DDD that does not require multiple injections.

Because DDD is a continuous process and because it is believed that some of the cytokines of interest both produce pain and degrade the ECM when present within the nucleus pulposus, it is desirable for the antagonist to remain within the nucleus pulposus as long as possible in a pharmaceutically effective amount. The half-life of the antagonist within the nucleus pulposus will depend upon many factors, including the size of the antagonist and its charge. In general, the larger the molecular weight of the antagonist, the more likely it is to remain contained by the annulus fibrosus portion of the disc.

When using a short half-life antagonist, it would be desirable for a relatively large dose of the antagonist to be administered transdiscally (i.e., into the disc). In this condition, quick depletion of the antagonist would not cause the antagonist to fall below therapeutically effective levels in the disc until an extended period of time has elapsed.

Although a large dose of the antagonist would be desirable in such instances, injecting a critical volume of water can increase pressure in the nucleus pulposus. Nociceptors present on the inner wall of the annulus fibrosus react to this increased pressure and produce pain. In some cases, the added amount could be as little as one cc by volume to produce pain. Accordingly, if a dilute concentration of an antagonist is added to the nucleus pulposus to provide a large dose, the resulting pressure increase caused by this added volume could be sufficient to cause acute pain.

For example, if it were determined that about 100 mg of an antagonist was needed to therapeutically effect a nucleus pulposus, and that antagonist was provided in concentrations of from about 30 to about 60 mg/ml, then at least about 1.5 ml of the antagonist would need to be injected into the nucleus pulposus in order to provide the desired therapeutic effect. However, when injecting volumes into the nucleus pulposus, it is desirable that the volume of drug delivered be no more than about 1 ml, preferably no more than about 0.5 ml (*i*.*e*., a maximum of 0.5 ml), more preferably between about 0.1 and about 0.3 ml. When injected in these smaller quantities, it is believed the added volume will not cause an appreciable pressure increase in the nucleus pulposus.

Accordingly, in some embodiments, the concentration of the antagonist in the administered formulation is at least about 100 mg/ml. When about 100 mg of the antagonist is needed to produce the desired therapeutic result, no more than about 1 ml of the drug need be injected. In some embodiments, the concentration of the antagonist in the administered drug is at least about 200 mg/ml. In this condition, no more than about 0.5 ml of the drug need be injected. In some embodiments, the concentration of the antagonist in the administered drug is at least about 500 mg/ml. In this condition, between about 0.03 to about 0.3 ml of the formulation need be injected.

The antagonist is provided in a sustained release device (*i.e*., sustained delivery device). The sustained release device is adapted to remain within the disc for a prolonged period and slowly release the antagonist contained therein to the surrounding environment. This mode of delivery allows an antagonist to remain in therapeutically effective amounts within the disc for a prolonged period.

In some embodiments, the antagonist is predominantly released from the sustained delivery device by its diffusion through the sustained delivery device (*e.g.*, through a polymer). In others, the antagonist is predominantly released from the sustained delivery device by the biodegradation of the sustained delivery device (*e*.*g*., biodegradation of a polymer).

In some embodiments, the sustained release device comprising a hydrogel comprises a bioresorbable material whose gradual erosion causes the gradual release of the antagonist to the disc environment. In some embodiments, the sustained release device comprises a bioresorbable polymer. In some embodiments, the bioresorbable polymer has a half-life of at least one month, more preferably at least two months, more preferably at least 6 months.

In some embodiments, the sustained delivery device comprises bioerodable macrospheres. The antagonist is preferably contained in a gelatin (or water or other solvent) within the capsule, and is released to the disc environment when the outer shell has been eroded. The device can include a plurality of capsules having outer shells of varying thickness, so that the sequential breakdown of the outer shells provides periodic release of the antagonist.

In some embodiments, the sustained release device provides controlled release. In some embodiments, it provides continuous release. In some embodiments, it provides intermittent release. In some embodiments, the sustained release device comprises a biosensor. Other release modes may also be used.

In some embodiments, the sustained delivery device comprises an inflammatory-responsive delivery system, such as one comprising bioerodable microspheres that are eroded by invading macrophages. This technology provides a high correspondence between physiologic inflammation of disc environment and the release of the antagonists into that environment. In one embodiment, the technology disclosed in Brown et al., Arthritis. Rheum. Dec., 41(12): 2185-95 (1998) is selected.

In some embodiments, the sustained delivery device comprises the devices disclosed in U.S. Patent No. 5,728,396 ("Peery").

In some embodiments, the sustained delivery device comprises a liposomal delivery system, such as that disclosed in WO 03/000190. Liposomes are small spheres whose walls are layers of lipids with water. As they form, liposomes entrap water and any water soluble solutes that are present. Because of this entrapping ability, they are useful as delivery systems. For the purposes of the present invention, a preferred embodiment includes the use of a multilamellar vesicle, and any naturally occurring phospholipid, such as dipalmitoylphosphatidylcholine (DPPC).

A liposome may be a vesicle having at least one lipid bilayer surrounding an inner liquid phase (a lipid bilayer surrounding either a liquid core or a liquid phase dispersed between it and another lipid bilayer). The liposome may have various structures such as multilamellar (MLVs), unilamellar (ULVs) and paucilamellar (PLVs) vesicles. The resulting structure of the liposome is dependent, in part, on the choice of materials forming the hydrophobic phase and the manufacturing parameters, such as temperature and incubation time.

Some liposomes comprise at least one amphiphilic bilayer-forming substance. The therapeutic substances contained therein may be contained either within the lipid bilayer or the hydrophilic compartments of the liposome. The amphiphilic bilayer-forming substance comprises both a hydrophilic and a lipophilic group and is capable of forming, either alone or in combination with other lipids, the bilayer of a liposome. The lipid can have single or multiple lipophilic side chains being either saturated or unsaturated in nature and branched or linear in structure. The amphiphilic bilayer-forming substance can be a phospoholipid or a ceramide.

In some embodiments, the sustained delivery device comprises a plurality (e.g., at least one hundred) of water-containing chambers, each chamber containing the antagonist. A chamber is defined by bilayer lipid membranes comprising synthetic duplicates of naturally occurring lipids. Release of the drug can be controlled by varying at least one of the aqueous excipients, the lipid components, and the manufacturing parameters. Preferably, the formulation comprises no more than 10% lipid. In some embodiments, the DEPOFOAM^{™} technology of Skyepharma PLC (London, United Kingdom) is selected.

In some embodiments, the sustained delivery device comprises a delivery system disclosed in U.S. Patent No. 5,270,300 ("Hunziker").

In some embodiments, the sustained delivery device comprises the co-polymer poly-DL-lactide-co-glycolide (PLG). Preferably, the formulation is manufactured by combining the antagonist or additional therapeutic agent, the co-polymer and a solvent to form a droplet, and then evaporating the solvent to form a microsphere. The plurality of microspheres are then combined in a biocompatible diluent. Preferably, the antagonist or additional therapeutic agent is released from the co-polymer by its diffusion through and by the biodegradation of the co-polymer. In some embodiments hereof, the PROLEASE^{™} technology of Alkermes (located in Cambridge, MA) is selected.

A "hydrogel" is a substance formed when an organic polymer (natural or synthetic) is set or solidified to create a three- dimensional open-lattice structure that entraps molecules of water or other solution to form a gel. The solidification can occur, *e.g*., by aggregation, coagulation, hydrophobic interactions, or cross-linking. The hydrogels are also biocompatible, *e*.*g*., not toxic, to substances suspended in the hydrogel.

A "hydrogel-antagonist composition" is a suspension of a hydrogel containing desired antagonist. The hydrogel-antagonist composition forms a uniform distribution of antagonist with a well-defined and precisely controllable density. Moreover, the hydrogel can support very large densities of antagonist. In addition, the hydrogel allows diffusion of nutrients to, and waste products away from, the endplates, which promotes tissue growth.

Hydrogels suitable for use in the present invention include water-containing gels, *i.e*., polymers characterized by hydrophilicity and insolubility in water. See, for instance, "Hydrogels", pages 458-459 in Concise Encyclopedia of Polymer Science and Engineering. Eds. Mark et al., Wiley and Sons (1990). Although their use is optional in the present invention, the inclusion of hydrogels is can be highly advantageous since they tend to possess a number of desirable qualities. By virtue of their hydrophilic, water-containing nature, hydrogels can assist with load bearing capabilities of the disc.

In one embodiment, the hydrogel is a fine, powdery synthetic hydrogel. Suitable hydrogels exhibit an optimal combination of such properties as compatibility with the matrix polymer of choice, and biocompatability. The hydrogel can include any one or more of the following: polysaccharides, proteins, polyphosphazenes, poly(oxyethylene)-poly(oxypropylene) block polymers, poly(oxyethylene)-poly(oxypropylene) block polymers of ethylene diamine, poly(acrylic acids), poly(methacrylic acids), copolymers of acrylic acid and methacrylic acid, poly(vinyl acetate), and sulfonated polymers.

In general, these polymers are at least partially soluble in aqueous solutions, *e.g*., water, or aqueous alcohol solutions that have charged side groups, or a monovalent ionic salt thereof There are many examples of polymers with acidic side groups that can be reacted with cations, *e.g*., poly(phosphazenes), poly(acrylic acids), and poly(methacrylic acids). Examples of acidic groups include carboxylic acid groups, sulfonic acid groups, and halogenated (preferably fluorinated) alcohol groups. Examples of polymers with basic side groups that can react with anions are poly(vinyl amines), poly(vinyl pyridine), and poly(vinyl imidazole).

In some embodiments, the sustained delivery device includes a polymer selected from the group consisting of PLA, PGA, PCL and mixtures thereof

If the half-life of the antagonist within the disc is relatively long, then it may be assumed that a relatively small dose of the antagonist can be administered into the disc. In this condition, the slow depletion of the antagonist would not cause the antagonist to fall below therapeutically effective levels in the disc until an extended period of time has elapsed.

In some embodiments in which antagonists have long half-lives within the disc space, the dose administered can be very small.

For example, if it is believed that an antagonist is effective when present in the range of about I to about 10 mg/kg or from about 1 to about 10 ppm (as is believed to be the case for the TNF antagonist REMICADE^{®} infliximab), and since a typical nucleus pulposus of a disc has a volume of about 3 ml (or 3 cc, or 3g), then only about 3 to about 30 ug of the antagonist need be administered to the disc in order to provide a long lasting effective amount of the antagonist. The formulation can be administered in an amount of less than 1 cc. As a point of reference, Tobinick discloses that at least 1 mg of cytokine antagonist should be administered perispinally in order to cure back pain. Similarly, Olmarker mixed 100 ml of a formulation comprising 1.11 mg/ml of a monoclonal antibody into 40 mg of an extracted nucleus pulposus, thereby producing a monoclonal antibody concentration of about 3 parts per thousand. The smaller amounts available by this route reduce the chances of deleterious side effects of the antagonist.

For example, if a clinician administered 0.3 ml of 60 mg/ml of an antagonist, such as REMICADE^{®} infliximab, into a 2.7 cc disc, this would produce an antagonist concentration in the disc of about 6 mg/ml, or 6 parts per thousand. Without wishing to be tied to a theory, if an antagonist (e.g., a HAAMMP) has the same half-life within a nucleus pulposus as it does when administered systemically (*i.e,* about 1 week), then the concentration of the antagonist would remain above about 10 ppm for about 9 weeks. Therefore, if another dose were needed, the clinician would only need to provide the second dose after about two months.

Therefore, in some embodiments, the antagonist is provided in a dose of less than about I mg, *e.g.,* less than about 0.5 mg, more preferably, less than about 0.1 mg, more preferably less than about 0.01 mg, more preferably less than about 0.001 mg. The smaller amounts available by this route reduce the chances of deleterious side effects of the antagonist. When the antagonist is a HSCA, then preferably, the HSCA provided in these smaller amounts is a TNF antagonist, more preferably it is REMICADE^{®} infliximab.

In some embodiments, the formulation of the present invention is administered directly into the disc through the outer wall of the annulus fibrosus, depositing the antagonist in the nucleus pulposus portion of the disc. In this condition, the fibrous nature of the annulus fibrosus that surrounds the nucleus pulposus will help keep the antagonist contained within the disc.

In some embodiments, the formulation of the present invention is injected into the disc through a small bore needle. In some embodiments, the needle has a bore of about 22 gauge or less, so that the possibilities of producing a herniation are mitigated. For example, the needle can have a bore of about 24 gauge or less, so that the possibilities of producing a herniation are even further mitigated.

If the volume of the direct injection of the formulation is sufficiently high so as to cause a concern of overpressurizing the nucleus pulposus, then it is preferred that at least a portion of the nucleus pulposus be removed prior to direct injection. In some embodiments, the volume of removed nucleus pulposus is substantially similar to the volume of the formulation to be injected. For example, the volume of removed nucleus pulposus can be within 80-120% of the volume of the formulation to be injected. In addition, this procedure has the added benefit of at least partially removing some degenerated disc from the patient.

In other embodiments, the formulation is delivered into the disc space through the endplate of an opposing vertebral body. This avenue eliminates the need to puncture the annulus fibrosus, and so eliminates the possibility of herniation.

In accordance with one embodiment of the invention, both the antagonist and at least one additional (e.g., second) therapeutic agent(s) are locally administered into the disc space. Because the HSCA is specific, it does not interfere with the locally administered additional therapeutic agent, and so each agent may independently work to provide therapy to the diseased disc.

More than one additional therapeutic agent can be administered. For example, there can be third, fourth and fifth therapeutic agents.

In some embodiments, the antagonist and additional therapeutic agent(s) (*i*.*e*., additional therapeutic substance) are administered simultaneously. In one embodiment, the antagonist and additional therapeutic agents are co-administered in a formulation comprising the antagonist and the additional therapeutic agent. In others, the antagonist(s) are administered first. In still others, the second therapeutic agent is administered first.

The additional therapeutic agent(s) can be one or more of the antagonists disclosed herein. For example, other compounds which may be added to the disc include, but are not limited to: vitamins and other nutritional supplements; hormones; glycoproteins; fibronectin; peptides and proteins; carbohydrates (both simple and/or complex); proteoglycans; oligonucleotides (sense and/or antisense DNA and/or RNA); bone morphogenetic proteins (BMPs); antibodies (for example, to infectious agents, tumors, drugs or hormones antiangiogenins; demineralized bone matrix (DBM); antibodies (for example, to infectious agents, tumors, or drugs); gene therapy reagents; and anti-cancer agents. If desired, substances such as pain killers and narcotics may also be admixed with the polymer for delivery and release to the disc space.

In some embodiments, growth factors are additional therapeutic agents. As used herein, the term "growth factors" encompasses any cellular product that modulates the growth or differentiation of other cells, particularly connective tissue progenitor cells. The growth factors that may be used in accordance with the present invention include, but are not limited to, members of the fibroblast growth factor family, including acidic and basic fibroblast growth factor (FGF-1 and -2) and FGF-4, members of the platelet-derived growth factor (PDGF) family, including PDGF-AB, PDGF-BB and PDGF-AA; EGFs, members of the insulin-like growth factor (IGF) family, including IGF-I and -II;, the TGF-β superfamily, including TGF-β1, 2 and 3 (including MAP-52), osteoid-inducing factor (OIF), angiogenin(s), endothelins, hepatocyte growth factor and keratinocyte growth factor; members of the bone morphogenetic proteins (BMPs) BMP-1; BMP-3; BMP-2; OP-1; BMP-2A, -2B, -4, -7 and -14; HBGF-1 and HBGF-2; growth differentiation factors (GDFs), members of the hedgehog family of proteins, including indian, sonic and desert hedgehog; ADMP-1; GDF-5; and members of the colony-stimulating factor (CSF) family, including CSF-1, G-CSF, and GM-CSF; and isoforms thereof.

In some embodiments, the growth factor is selected from the group consisting of TGF-β, bFGF, and IGF-1. These growth factors are believed to promote regeneration of the nucleus pulposus, or stimulate proliferation and/or differentiation of chondrocytes, as well as ECM secretion. In one embodiment, the growth factor is TGF-β. More preferably, TGF-β is administered in an amount of between about 10 ng/ml and about 5000 ng/ml, for example, between about 50 ng/ml and about 500 ng/ml, *e.g*., between about 100 ng/ml and about 300 ng/ml.

In some embodiments, platelet concentrate is provided as an additional therapeutic agent. In one embodiment, the growth factors released by the platelets are present in an amount at least two-fold (*e.g*., four-fold) greater than the amount found in the blood from which the platelets were taken. In some embodiments, the platelet concentrate is autologous. In some embodiments, the platelet concentrate is platelet rich plasma (PRP). PRP is advantageous because it contains growth factors that can restimulate the growth of the ECM, and because its fibrin matrix provides a suitable scaffold for new tissue growth.

In one embodiment, the additional therapeutic agent is an antagonist of NO synthase. In one embodiment, the antagonist is L-NIL and N^{G} - monomethyl-L-arginine. In one embodiment, the additional therapeutic agent is an antagonist of PLA₂. In one embodiment, the additional therapeutic agent is a high specificity anti-proliferative agent, such as a high specificity anti-proliferative agent comprising rapamycin, a cdk inhibitor, a statin, and an anti-oxidant. In yet another embodiment, the anti-oxidant comprises a super oxide dismutase.

In one embodiment, the additional therapeutic agent is a highly specific apoptosis inhibitor selected from the group consisting of an EPO mimetic peptide and an EPO mimetic antibody fusion protein. In one embodiment, the additional therapeutic agent is a highly specific apoptosis inhibitor selected from the group consisting of IGF-I and IGF-II. In one embodiment, the additional therapeutic agent is a highly specific caspase inhibitor. In one embodiment, the additional therapeutic agent comprises glycosaminoglycans.

In one embodiment, the formulation further comprises a liposomal delivery system.

In some embodiments, the formulation comprises a suitable biocompatible carrier such as saline. In some embodiments, the carrier is selected from the carriers disclosed in US Patent No. 6,277,969 ("Le").

In some embodiments, the formulation is administered through a drug pump.

In some preferred embodiments, the antagonist is combined in the formulation with a viscosupplement. The viscosupplement has characteristics substantially similar to that of natural healthy nucleus pulposus ECM.

Preferably, the viscosupplement comprises glycosaminoglycans (GAGS). GAGS are biopolymers consisting of repeating polysaccharide units, and are present in nature on the cell surface as well as in the extracellular matrix of animals. GAGS are long unbranded polysaccharides containing a repeating disaccharide unit. The disaccharide unit contains either of two modified sugars, N-acetylgalactosaimne or N-acetylglucosamine and a uronic acid such as glucuronate or iduronate. GAGS are highly negatively charged molecules, with extended conformation that imparts high viscosity to the solution. In addition, to high viscosity, GAGS routinely possess low compressability, which makes these molecules ideal for a lubricating fluid in the joints. At the same time, their rigidity provides structural integrity to cells and provides passageways between cells, allowing for cell migration.

Recent work has shown that plasmid DNA will not elicit an inflammatory response as does the use of viral vectors. Genes encoding cartilage (anabolic) agents such as BMP may be efficacious if injected into the joint. In addition, overexpression of any of the growth factors provided herein or other agents such as a tissue inhibitor of MMPs (TIMP) that would limit local MMP activity would have positive effects on chondrocyte and ECM protection. In some embodiments, the TIMP is selected from the group consisting of TIMP-1 and TIMP-2. In some embodiments, the TIMP is autologous and is concentrated by filtration, centrifugation or by immuno-attachment processes. In other embodiments, the TIMP is manufactured recombinantly, and is preferably present in a concentration of at least 1000 times that found in the patient. In one embodiment, the plasmid contains the genetic code for human TGF-β or EPO.

Hyaluronic acid (HA) is a high molecular weight polysaccharide of N-acetyl glucosaime and glucuronic acid molecules that is naturally occurring in all mammals in a variety of tissue and some bacterial species. For the purposes of this invention, HA includes any derivatives such as hyaluronan and hyaluronic acid itself with H+ ion attached to the COO- group, and salts of hyaluronic acid whereby another positive ion replaces the H+ ion, as for examples, with Na+ which forms sodium hyaluronate. Also included in the definition of HA is any physically or chemically cross-linked hyaluronic acid or derivative. HA is unique among the GAGS in that it does not contain any sulphate and is not found covalently attached to proteins as a proteoglycan. HA polymers are very large, with molecular weights of between about 100,000 and 10,000,000, and can displace a large volume of water. For the purposes of the present invention, a preferred embodiment includes a non-cross linked HA with a molecular weight of 0.5 - 10 M Dalton.

Preferably, the viscosupplement is selected from the group consisting of hyaluronic acid and hyaluronate (either cross-linked or uncross-linked). In some embodiments, the viscosupplement is ARTHREASE^{™} (DePuy Ltd., Leeds, U.K.). In some embodiments, the viscosupplement is a hyaluronic acid selected from the hyaluronic acids disclosed in U.S. Patent no. 6,645,945 (U.S. Serial No. 09/298,539, entitled "Method of Treating Diseased, Injured or Abnormal Cartilage with Hyaluronic Acid and Growth Factors" (Radomsky *et al*.)).

Recent work has shown that plasmid DNA will not elicit an inflammatory response as does the use of viral vectors. Genes encoding cartilage (anabolic) agents such as BMP may be efficacious if injected into the joint. In addition, overexpression of any of the growth factors provided herein or other agents such as TIMP which would limit local MMP activity would have positive effects on chondrocyte and ECM protection. In one embodiment, the plasmid contains the genetic code for human TGF-β or EPO.

Because the causes of low back pain may be myriad, and because of the significant cost of some antagonists, it would be useful for a clinician to first perform a diagnostic test in order to confirm that the targeted disc in fact possesses high levels of the targeted proteins (e.g., proinflammatory cytokines and/or MMPs) prior to providing the injection.

In one embodiment, the diagnostic test comprises a non-invasive diagnostic test comprising, for example, using an MRI. In some embodiments, the MRI is able to quantify the aggrecans levels within the disc.

In one embodiment, the clinician would first perform a discogram in order to identify which disc or discs are responsible for the patient's low back pain. Next, the clinician would perform an invasive or non-invasive test upon the targeted disc in order to confirm the presence of or quantify the level of the proinflammatory cytokines and/or MMPs.

In one embodiment, the diagnostic test comprises an invasive test in which a portion of the disc is removed and analysed. In some embodiments, the clinician removes a portion of the nucleus pulposus. In some embodiments, the clinician removes a portion of the annulus fibrosus. Preferably, the removed material is a portion of the nucleus pulposus. The presence of proinflammatory cytokines and/or MMPs in the removed material may detected by procedures including, but not limited, to electrophoresis, or an enzyme-linked immunoabsorbent assay (ELISA) (as per Burke, Br. JBJS, 84-B(2), 2002).

In some embodiments, the diagnostic methods disclosed in U.S. Patent No. 6,277,969 ("Le") are selected. In these methods, high specificity anti- TNF-α compounds are used as diagnostic tools for detecting TNF-α in the patient known or suspected to have a high level of TNF-α.

After determining the levels of the different proinflammatory cytokines and/or MMPs in the degenerating disc, the clinician will preferably proceed to compare these diagnosed levels against pre-determined levels of the protein or proteins (such as cytokines or MMPs). If the diagnosed level of the proinflammatory cytokines and/or MMPs exceeds the pre-determined level, then the clinician may conclude that these higher levels are causing unwanted inflammatory action and proceed to directly inject a desired antagonist (*e.g*., a high specificity p38 kinase inhibitor) into the disc capable of inhibiting the targeted protein.

In some embodiments, a bioMEMS device containing a "lab on a chip" used in the diagnostic test.

In some embodiments, the predetermined level for an interleukin is at least 100 pg/ml. In some embodiments, the predetermined level for IL-6 is at least 250 pg/ml. In some embodiments, the predetermined level for IL-8 is at least 500 pg/ml. in some embodiments, the predetermined level for PGE2 is at least 1000 pg/ml. In some embodiments, the predetermined level for TNF-α is at least 500 pg/ml. In others, the predetermined level for TNF-α is at least 20 pg/ml, more preferably at least 30 pg/ml, more preferably at least 50 pg/ml, more preferably at least I ng/ml. In others, the predetermined level for TNF-α is at least 1 ng/disc, and in others, it is at least 1000 pg/disc.

It would also be useful to be able to determine whether directly administering the therapeutic substances of the present invention was, in fact, efficacious. Accordingly, one can measure the level of cytokines or MMPs remaining in the disc after administration.

Comparative examples set out below include details of formulations which do not necessarily have all of the features of the invention. They are included to facilitate understanding of the invention.

### COMPARATIVE EXAMPLE I

This non-limiting prophetic example describes how to transdiscally administer a formulation comprising an HAAMMP and saline into a nucleus pulposus of a degenerating disc.

First, a clinician uses a diagnostic test to verify that a particular disc within a patient has high levels of MMPs.

Next, the clinician provides a local anesthetic (such as 5 ml lidocaine) to the region dorsal of the disc of concern to reduce subcutaneous pain.

Next, the clinician punctures the skin of the patient dorsal the disc of concern with a relatively large (*e.g*., 18-19 gauge) needle having a stylet therein, and advances the needle through subcutaneous fat and dorsal sacrolumbar ligament and muscles to the outer edge of the intervertebral disc.

Next, the stylet is removed from the needle.

Next, the clinician receives a syringe having a smaller gauge needle adapted to fit within the larger gauge needle. This needle is typically a 22 or 24 gauge needle. The barrel of the syringe contains the formulation of the present invention.

The formulation contains an HAAMMP, and has an HAAMMP concentration of between about 30 mg/ml and about 60 mg/ml.

Next, the physician advances the smaller needle co-axially through the larger needle and past the distal end of the larger needle, thereby puncturing the annulus fibrosus. The smaller needle is then further advanced into the center of the nucleus pulposus. Finally, the clinician depresses the plunger of the syringe, thereby injecting between about 0.1 and I ml of the formulation into the nucleus pulposus.

### COMPARATIVE EXAMPLE II

This non-limiting prophetic example is substantially similar to that of Example I, except that the formulation comprises a sustained release device comprising the co-polymer poly-DL-lactide-co-glycolide (PLG). The formulation contains HAAMMP as the antagonist, and has an HAAMMP concentration of between about 30 mg/ml and about 60 mg/ml.

### COMPARATIVE EXAMPLE III

This non-limiting prophetic example describes how to transdiscally administer a formulation comprising a HSCA and saline into a nucleus pulposus of a degenerating disc.

First, a clinician uses a diagnostic test to verify that a particular disc within a patient has high levels of a particular pro-inflammatory cytokine.

Next, the clinician provides a local anesthetic (such as 5 ml lidocaine) to the region dorsal of the disc of concern to reduce subcutaneous pain.

Next, the clinician punctures the skin of the patient dorsal the disc of concern with a relatively large (*e.g*., 18-19 gauge) needle having a stylet therein, and advances the needle through subcutaneous fat and dorsal sacrolumbar ligament and muscles to the outer edge of the intervertebral disc.

Next, the stylet is removed from the needle.

Next, the clinician receives a syringe having a smaller gauge needle adapted to fit within the larger gauge needle. This needle is typically a 22 or 24 gauge needle. The barrel of the syringe contains the formulation of the present invention.

The formulation contains REMICADE^{®} infliximab, and has an infliximab concentration of between about 30 mg/ml and about 60 mg/ml.

Next, the physician advances the smaller needle co-axially through the larger needle and past the distal end of the larger needle, thereby puncturing the annulus fibrosus. The smaller needle is then further advanced into the center of the nucleus pulposus. Finally, the clinician depresses the plunger of the syringe, thereby injecting between about 0.1 and 1 ml of the formulation into the nucleus pulposus.

### COMPARATIVE EXAMPLE IV

This non-limiting prophetic example describes how to administer transdiscally a formulation comprising a cycline compound into a nucleus pulposus of a degenerating disc.

First, a clinician uses a diagnostic test to verify that a particular disc within a patient has high levels of a particular pro-inflammatory cytokine and/or MMPs.

Next, the clinician provides a local anesthetic (such as 5 ml lidocaine) to the region dorsal of the disc of concern to reduce subcutaneous pain.

Next, the clinician punctures the skin of the patient dorsal to the disc of concern with a relatively large (*e*.*g*., 18-19 gauge) needle having a stylet therein, and advances the needle through subcutaneous fat and dorsal sacrolumbar ligament and muscles to the outer edge of the intervertebral disc.

Next, the stylet is removed from the needle.

In one embodiment, the portion of the nucleus pulposus is removed prior to administering the cycline compound.

Next, the clinician receives a syringe having a smaller gauge needle adapted to fit within the larger gauge needle. This needle is typically a 22 or 24 gauge needle. The barrel of the syringe contains the formulation of the present invention.

The formulation contains about 50 mg/ml of doxycycline.

Next, the physician advances the smaller needle co-axially through the larger needle and past the distal end of the larger needle, thereby puncturing the annulus fibrosus. The smaller needle is then further advanced into the center of the nucleus pulposus. Finally, the clinician depresses the plunger of the syringe, thereby injecting between about 0.1 and about 1 ml of the formulation into the nucleus pulposus.

### COMPARATIVE EXAMPLE V

This non-limiting prophetic example is substantially similar to that of Example I, except that the formulation comprises a sustained release device comprising the co-polymer poly-DL-lactide-co-glycolide (PLG). The formulation contains doxycycline.

### EXAMPLE

This non-limiting prophetic example is substantially similar to that of Comparative Example III, except that the formulation comprises a sustained release device comprising the co-polymer poly-DL-lactide-co-glycolide (PLG). The formulation contains infliximab as the antagonist, and has an infliximab concentration of between about 30 mg/ml and about 60 mg/ml.

## Claims

1. A formulation for use in a method of treating degenerative disc disease by injection of the formulation into the nucleus pulposus, in which the formulation comprises a high specificity cytokine antagonist which inhibits tumour necrosis factor-α (TNF-α) and a sustained release device comprising a hydrogel.

2. A formulation for use in a method of treating degenerative disc disease as claimed in claim 1, in which the antagonist is water insoluble or substantially water insoluble.

3. A formulation for use in a method of treating degenerative disc disease as claimed in claim 1, which includes at least one additional therapeutic agent.

4. A formulation for use in a method of treating degenerative disc disease as claimed in claim 1, which is administered in the degenerative disc disease treatment method in an amount effective to reduce pain.

5. A formulation for use in a method of treating degenerative disc disease as claimed in claim 1, which is administered in the degenerative disc disease treatment method in an amount effective to inhibit degradation of an extracellular matrix of the nucleus pulposus.

6. A formulation for use in a method of treating degenerative disc disease as claimed in claim 1. which is administered in the degenerative disc disease treatment method in a dosage to produce a local tissue concentration of between about 5 µg.kg⁻¹ and about 50 µg.kg⁻¹.

7. A formulation for use in a method of treating degenerative disc disease as claimed in claim 1, which is administered in the degenerative disc disease treatment method in an amount of less than about 1 cc.

8. A formulation for use in a method of treating degenerative disc disease as claimed in claim 1, in which the antagonist is present in an amount of at least about 100 mg.ml⁻¹.

9. A formulation for use in a method of treating degenerative disc disease as claimed in claim 1, in which the antagonist is present in an amount of no more than about 0.5 mg.

10. A formulation for use in a method of treating degenerative disc disease as claimed in claim 1, which includes a growth factor present in an amount effective to repair disc tissue.

11. A formulationfor use in a method of treating degenerative disc disease as claimed in claim 1, which is administered in the degenerative disc disease treatment method through a drug pump.

12. A formulationfor use in a method of treating degenerative disc disease as claimed in claim 1, which is administered in the degenerative disc disease treatment method through a needle.

13. A formulationfor use in a method of treating degenerative disc disease as claimed in claim 1, which is administered in the degenerative disc disease treatment method in a volume of between about 0.03 ml and about 0.3 ml.

14. A formulation asfor use in a method of treating degenerative disc disease claimed in claim 1, in which the sustained release device provides controlled release.

15. A formulationfor use in a method of treating degenerative disc disease as claimed in claim 1, in which the sustained release device provides continuous release.

16. A formulationfor use in a method of treating degenerative disc disease as claimed in claim 1, in which the sustained release device provides intermittent release.

17. A formulationfor use in a method of treating degenerative disc disease as claimed in claim 1, in which the sustained release device comprises a biosensor.

18. A formulationfor use in a method of treating degenerative disc disease as claimed in claim 1, in which the sustained release device comprises microspheres.

19. A formulationfor use in a method of treating degenerative disc disease as claimed in claim 1, which further comprises a growth factor present in an amount effective to repair disc tissue.

20. A formulationfor use in a method of treating degenerative disc disease as claimed in claim 19. in which the growth factor is a TGF-β3.

21. A formulationfor use in a method of treating degenerative disc disease as claimed in claim 19, in which the growth factor is provided by platelet concentrate.

22. A formulationfor use in a method of treating degenerative disc disease as claimed in claim 1, which includes glycosaminoglycans.

23. A formulationfor use in a method of treating degenerative disc disease as claimed in claim 1, which includes a buffer.

24. A formulationfor use in a method of treating degenerative disc disease as claimed in claim 1, in which the antagonist is a monoclonal antibody.

25. A formulationfor use in a method of treating degenerative disc disease as claimed in claim 24, in which the monoclonal antibody is infliximab.

26. A formulationfor use in a method of treating degenerative disc disease as claimed in claim 1, in which the antagonist is etanercept.

## Patentansprüche

1. Formulierung zur Verwendung in einem Verfahren zum Behandeln von degenerativer Bandscheibenerkrankung durch Injizieren der Formulierung in den Nucleus pulposus, wobei die Formulierung einen Zytokin-Antagonisten mit hoher spezifischer Wirksamkeit aufweist, der den Tumor-Nekrosefaktor a (TNF-α) unterdrückt, und eine Einheit zur fortwährenden Freigabe, die ein Hydrogel aufweist.

2. Formulierung zur Verwendung bei einem Verfahren zum Behandeln von degenerativer Bandscheibenerkrankung nach Anspruch 1, bei dem der Antagonist in Wasser unlöslich oder im Wesentlichen in Wasser unlöslich ist.

3. Formulierung zur Verwendung bei einem Verfahren zum Behandeln von degenerativer Bandscheibenerkrankung nach Anspruch 1, die wenigstens einen zusätzlichen therapeutischen Wirkstoff umfasst.

4. Formulierung zur Verwendung bei einem Verfahren zum Behandeln von degenerativer Bandscheibenerkrankung, die bei dem Verfahren zum Behandeln von degenerativer Bandscheibenerkrankung in einer Menge verabreicht wird, die wirksam ist, um Schmerzen zu verringern.

5. Formulierung zur Verwendung bei einem Verfahren zum Behandeln von degenerativer Bandscheibenerkrankung, die bei dem Verfahren zum Behandeln von degenerativer Bandscheibenerkrankung in einer Menge verabreicht wird, die wirksam ist, um den Abbau einer extrazellularen Matrix des Nucleus pulposus zu verhindern.

6. Formulierung zur Verwendung bei einem Verfahren zum Behandeln von degenerativer Bandscheibenerkrankung nach Anspruch 1, die bei dem Verfahren zum Behandeln von degenerativer Bandscheibenerkrankung in einer Dosierung verabreicht wird, um eine lokale Konzentration im Gewebe zwischen ungefähr 5 µg.kg⁻¹ und ungefähr 50 µg.kg⁻¹ zu erzeugen.

7. Formulierung zur Verwendung bei einem Verfahren zum Behandeln von degenerativer Bandscheibenerkrankung nach Anspruch 1, die bei dem Verfahren zur Behandlung von degenerativer Bandscheibenerkrankung in einer Menge von weniger als ungefähr 1 cm³ verabreicht wird.

8. Formulierung zur Verwendung bei einem Verfahren zum Behandeln von degenerativer Bandscheibenerkrankung nach Anspruch 1, bei der der Antagonist in einer Menge von wenigstens ungefähr 100 mg.m1⁻¹ vorhanden ist.

9. Formulierung zur Verwendung bei einem Verfahren zum Behandeln von degenerativer Bandscheibenerkrankung nach Anspruch 1, bei der der Antagonist in einer Menge von nicht mehr als ungefähr 0.5 mg vorhanden ist.

10. Formulierung zur Verwendung bei einem Verfahren zum Behandeln von degenerativer Bandscheibenerkrankung nach Anspruch 1, die einen Wachstumsfaktor umfasst, der in einer Menge vorhanden ist, die wirksam ist, Bandscheibengewebe wiederherzustellen.

11. Formulierung zur Verwendung bei einem Verfahren zum Behandeln von degenerativer Bandscheibenerkrankung nach Anspruch 1, die bei dem Verfahren zum Behandeln von degenerativer Bandscheibenerkrankung durch eine Arzneimittelpumpe verabreicht wird.

12. Formulierung zur Verwendung bei einem Verfahren zum Behandeln von degenerativer Bandscheibenerkrankung nach Anspruch 1, die bei dem Verfahren zum Behandeln von degenerativer Bandscheibenerkrankung durch eine Nadel verabreicht wird.

13. Formulierung zur Verwendung bei einem Verfahren zum Behandeln von degenerativer Bandscheibenerkrankung nach Anspruch 1, die bei dem Verfahren zum Behandeln von degenerativer Bandscheibenerkrankung in einem Volumen zwischen ungefähr 0.03 ml und ungefähr 0.3 ml verabreicht wird.

14. Formulierung zur Verwendung bei einem Verfahren zum Behandeln von degenerativer Bandscheibenerkrankung nach Anspruch 1, bei der die Einheit für fortwährende Freigabe für gesteuerte Freigabe sorgt.

15. Formulierung zur Verwendung bei einem Verfahren zum Behandeln von degenerativer Bandscheibenerkrankung nach Anspruch 1, bei der die Einheit für fortwährende Freigabe für eine kontinuierliche Freigabe sorgt.

16. Formulierung zur Verwendung bei einem Verfahren zum Behandeln von degenerativer Bandscheibenerkrankung nach Anspruch 1, bei der die Einheit für fortwährende Freigabe für unterbrochene Freigabe sorgt.

17. Formulierung zur Verwendung bei einem Verfahren zum Behandeln von degenerativer Bandscheibenerkrankung nach Anspruch 1, bei der die Einheit für fortwährende Freigabe einen Biosensor aufweist.

18. Formulierung zur Verwendung bei einem Verfahren zum Behandeln von degenerativer Bandscheibenerkrankung nach Anspruch 1, bei der die Einheit für fortwährende Freigabe Mikrokügelchen aufweist.

19. Formulierung zur Verwendung bei einem Verfahren zum Behandeln von degenerativer Bandscheibenerkrankung nach Anspruch 1, die weiter einen Wachstumsfaktor aufweist, der in einer Menge vorhanden ist, die wirksam ist, um Bandscheibengewebe wiederherzustellen.

20. Formulierung zur Verwendung bei einem Verfahren zum Behandeln von degenerativer Bandscheibenerkrankung nach Anspruch 19, bei der der Wachstumsfaktor ein TGF-β ist.

21. Formulierung zur Verwendung bei einem Verfahren zum Behandeln von degenerativer Bandscheibenerkrankung nach Anspruch 19, bei der der Wachstumsfaktor durch Plättchenkonzentrat zur Verfügung gestellt wird.

22. Formulierung zur Verwendung bei einem Verfahren zum Behandeln von degenerativer Bandscheibenerkrankung nach Anspruch 1, die Glykosaminglykane umfasst.

23. Formulierung zur Verwendung bei einem Verfahren zum Behandeln von degenerativer Bandscheibenerkrankung nach Anspruch 1, die einen Puffer umfasst,

24. Formulierung zur Verwendung bei einem Verfahren zum Behandeln von degenerativer Bandscheibenerkrankung nach Anspruch 1, bei der der Antagonist ein monoklonaler Antikörper ist.

25. Formulierung zur Verwendung bei einem Verfahren zum Behandeln von degenerativer Bandscheibenerkrankung nach Anspruch 24, bei der der monoklonale Antikörper Infliximab ist.

26. Formulierung zur Verwendung bei einem Verfahren zum Behandeln von degenerativer Bandscheibenerkrankung nach Anspruch 1, bei der der Antagonist Etanercept ist.

## Revendications

1. Formulation pour une utilisation dans une méthode de traitement d'une maladie discale dégénérative par injection de la formulation dans le noyau gélatineux, dans laquelle la formulation comprend un antagoniste de la cytokine de forte spécificité qui inhibe le facteur de nécrose tumorale α (TNF-α) et un dispositif à libération prolongée comprenant un hydrogel.

2. Formulation pour une utilisation dans une méthode de traitement d'une maladie discale dégénérative selon la revendication 1, dans laquelle l'antagoniste est insoluble dans l'eau ou est essentiellement insoluble dans l'eau.

3. Formulation pour une utilisation dans une méthode de traitement d'une maladie discale dégénérative selon la revendication 1, qui inclut au moins un autre agent thérapeutique.

4. Formulation pour une utilisation dans une méthode de traitement d'une maladie discale dégénérative selon la revendication 1, qui est administrée dans la méthode de traitement d'une maladie 0discale dégénérative en une quantité efficace pour réduire la douleur.

5. Formulation pour une utilisation dans une méthode de traitement d'une maladie discale dégénérative selon la revendication 1, qui est administrée dans la méthode de traitement d'une maladie discale dégénérative en une quantité efficace pour inhiber la dégradation d'une matrice extracellulaire du noyau gélatineux.

6. Formulation pour une utilisation dans une méthode de traitement d'une maladie discale dégénérative selon la revendication 1, qui est administrée dans la méthode de traitement d'une maladie discale dégénérative en une dose permettant de produire une concentration dans le tissu local d'environ 5 µg.kg⁻¹ à environ 50 µg.kg⁻¹.

7. Formulation pour une utilisation dans une méthode de traitement d'une maladie discale dégénérative selon la revendication 1, qui est administrée dans la méthode de traitement d'une maladie discale dégénérative en une quantité inférieure à environ 1 cm³,

8. Formulation pour une utilisation dans une méthode de traitement d'une maladie discale dégénérative selon la revendication 1, dans laquelle l'antagoniste est présent en une quantité d'au moins environ 100 mg.ml⁻¹.

9. Formulation pour une utilisation dans une méthode de traitement d'une maladie discale dégénérative selon la revendication 1, dans laquelle l'antagoniste est présent en une quantité nordi supérieure à environ 0,5 mg.

10. Formulation pour une utilisation dans une méthode de traitement d'une maladie discale dégénérative selon la revendication 1, qui inclut un facteur de croissance présent en une quantité efficace pour réparer le tissu discal.

11. Formulation pour une utilisation dans une méthode de traitement d'une maladie discale dégénérative selon la revendication 1, qui est administrée dans la méthode de traitement d'une maladie discale dégénérative par l'intermédiaire d'une pompe à médicament.

12. Formulation pour une utilisation dans une méthode de traitement d'une maladie discale dégénérative selon la revendication 1, qui est administrée dans la méthode de traitement d'une maladie discale dégénérative par l'intermédiaire d'une aiguille.

13. Formulation pour une utilisation dans une méthode de traitement d'une maladie discale dégénérative selon la revendication 1, qui est administrée dans la méthode de traitement d'une maladie discale dégénérative en un volume d'environ 0,03 ml à environ 0,3 ml.

14. Formulation pour une utilisation dans une méthode de traitement d'une maladie discale dégénérative selon la revendication 1, dans laquelle le dispositif à libération prolongée offre une libération contrôlée.

15. Formulation pour une utilisation dans une méthode de traitement d'une maladie discale dégénérative selon la revendication 1, dans laquelle le dispositif à libération prolongée offre une libération continue.

16. Formulation pour une utilisation dans une méthode de traitement d'une maladie discale dégénérative selon la revendication 1, dans laquelle le dispositif à libération prolongée offre une libération intermittente.

17. Formulation pour une utilisation dans une méthode de traitement d'une maladie discale dégénérative selon la revendication 1, dans laquelle le dispositif à libération prolongée comprend un biocapteur.

18. Formulation pour une utilisation dans une méthode de traitement d'une maladie discale dégénérative selon la revendication 1, dans laquelle le dispositif à libération prolongée comprend des microsphères.

19. Formulation pour une utilisation dans une méthode de traitement d'une maladie discale dégénérative selon la revendication 1, qui comprend en outre un facteur de croissance présent en une quantité efficace pour réparer un tissu discal.

20. Formulation pour une utilisation dans une méthode de traitement d'une maladie discale dégénérative selon la revendication 19, dans laquelle le facteur de croissance est un TGF-β.

21. Formulation pour une utilisation dans une méthode de traitement d'une maladie discale dégénérative selon la revendication 19, dans laquelle le facteur de croissance est fourni par un concentré de plaquettes.

22. Formulation pour une utilisation dans une méthode de traitement d'une maladie discale dégénérative selon la revendication 1, qui inclut des glycosaminoglycanes.

23. Formulation pour une utilisation dans une méthode de traitement d'une maladie discale dégénérative selon la revendication 1, qui inclut un tampon.

24. Formulation pour une utilisation dans une méthode de traitement d'une maladie discale dégénérative selon la revendication 1, dans laquelle antagoniste est un anticorps monoclonal.

25. Formulation pour une utilisation dans une méthode de traitement d'une maladie discale dégénérative selon la revendication 1, dans laquelle l'anticorps monoclonal est l'infliximab.

26. Formulation pour une utilisation dans une méthode de traitement d'une maladie discale dégénérative selon la revendication 1, dans laquelle l'antagoniste est l'étanercept.
